Europäisches Patentamt

**European Patent Office**

**Office européen des brevets**

(19)

(11) Veröffentlichungsnummer: **0 069 156 B1**

(12) **EUROPÄISCHE PATENTSCHRIFT**

(45) Veröffentlichungstag der Patentschrift:
29.05.85

(51) Int. Cl.⁴: **A 01 N 1/02** // A61F2/00

(21) Anmeldenummer: 81105117.6

(22) Anmeldetag: 02.07.81

(54) Verfahren zur Herstellung von Skleroproteintransplantaten mit erhöhter biologischer Stabilität.

(43) Veröffentlichungstag der Anmeldung:
12.01.83 Patentblatt 83/2

(73) Patentinhaber: **Intermedicat GmbH, Gerliswilstrasse 45, CH-6020 Emmenbrücke (CH)**

(45) Bekanntmachung des Hinweises auf die Patenterteilung:
29.05.85 Patentblatt 85/22

(72) Erfinder: **Werner, Heinz-Helmut, Dr., Am Steig 2, D-3508 Melsungen (DE)**

(84) Benannte Vertragsstaaten:
**AT BE CH DE FR GB IT LI LU NL SE**

(74) Vertreter: **von Kreisler, Alek, Dipl.-Chem. et al, Deichmannhaus am Hauptbahnhof, D-5000 Köln 1 (DE)**

(56) Entgegenhaltungen:
**DE - A - 2 004 553**
**DE - A - 2 024 341**
**GB - A - 994 275**
**GB - A - 1 018 288**

**Die Akte enthält technische Angaben, die nach dem Eingang der Anmeldung eingereicht wurden und die nicht in dieser Patentschrift enthalten sind.**

BUNDESDRUCKEREI BERLIN

## Beschreibung

Es ist bekannt, daß einige Skleroproteine wie z. B. Kollagen, Keratin, Elastin bei Mensch und Tier sowohl homolog als auch heterolog transplantiert werden können, um bestehende Defekte von körpereigenem Gewebe auszufüllen. Der Empfängerorganismus kann dabei je nach Art des Implantates mehr oder weniger gut erkennen, daß hier ein fremdes Eiweiß vorliegt. In der Regel kommt es dann zum Abbau des implantierten Stoffes. In einigen Fällen verläuft der Abbau des Implantates so langsam, daß der Körper gleichzeitig schon wieder neues Bindegewebe aufzubauen imstande ist. Hier spricht man dann vom Leitschienenprinzip, wie es z. B. in der DE-A-2 004 553.8 beschrieben ist.

Es ist einleuchtend, daß das Leitschienenprinzip versagen muß, wenn der Aufbau langsamer verläuft als der Abbau. Es ist daher in der Regel eine hohe Resistenz gegen die Abbaumaßnahmen des Körpers wünschenswert. Die folgende Literaturauswahl zeigt, welchen Stellenwert man der Implantation von Skleroproteinen in der Medizin beimessen muß. Vor allem die lyophilisierte harte Hirnhaut des Menschen ist als Transplantat vorwiegend in der Neurochirurgie kaum noch wegzudenken. Aber auch andere Kollagene, die vom Tier gewonnen wurden, spielen eine gewisse Rolle.

Verwiesen sei auf die Firmenbroschüre der Firma B. Braun Melsungen AG »Lyodura® zum homoiplastischen Ersatz von Körperstrukturen« aus dem Jahre 1978 und die darin genannten sehr zahlreichen Literaturstellen.

Als Aufbereitungsverfahren für Transplantate aus Skleroproteinen kommen heute im wesentlichen die Gefriertrocknung und die Acetontrocknung zur Anwendung.

Die biologische Stabilität ist dabei häufig unbefriedigend, vor allem beim Ersatz mechanisch beanspruchter Organe wie z. B. Fascien, Bänder und Sehnen. Nimmt die Stabilität des Implantates im Körper schneller ab als neue körpereigene Strukturen durch die Wundheilung aufgebaut werden können, kann der angestrebte Erfolg des operativen Eingriffes nicht mehr erreicht werden und der Patient erleidet entsprechende Schäden.

Die Erfindung stellt sich die Aufgabe, ein Verfahren zu schaffen, bei dem die biologische Stabilität von Skleroproteintransplantaten verbessert wird.

Bei experimentellen Untersuchungen wurde nun überraschend gefunden, daß durch ein modifiziertes Trocknungsverfahren die Resistenz der Skleroproteine gegen biologischen Abbau wesentlich gesteigert werden kann.

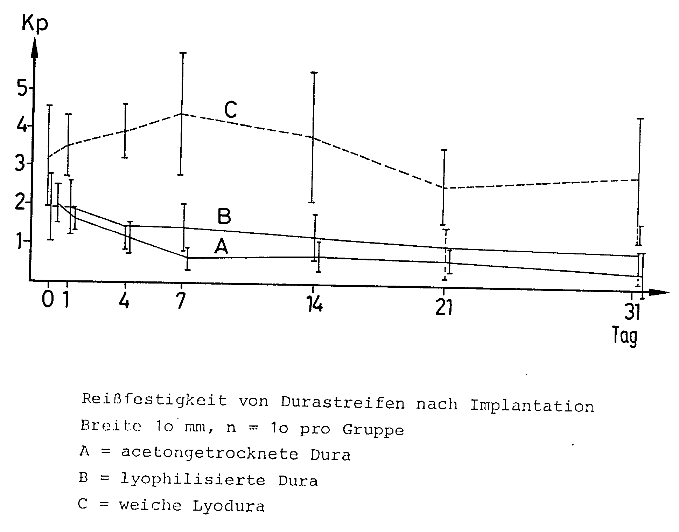

Aus der Zeichnung ist beispielhaft ersichtliche, welche Wirkung durch das erfindungsgemäße Verfahren erzielt werden kann.

Die Abbildung zeigt den Verlauf der Reißfestigkeit von 10 mm breiten Streifen harter Hirnhaut des Menschen nach Einpflanzung unter die Rückenhaut von Kaninchen. Hierbei wurden 210 solcher Streifen implantiert und zu verschiedenen Zeitpunkten nach der Operation wieder entnommen, um die dann noch vorhandene Reißfestigkeit zu messen, die Kurve A wurde mit acetongetrockneter Hirnhaut ermittelt, Kurve B mit gefriergetrockneter Hirnhaut und Kurve C mit Hirnhaut, die nach dem Verfahren der Erfindung hergestellt worden war.

Während zwischen den Verfahren Acetontrocknung und Gefriertrocknung kein signifikanter Unterschied in der Reißfestigkeit über den Beobachtungszeitraum von 31 Tagen hinweg gefunden wurde, wies das gemäß der Erfindung hergestellte Material eine Steigerung der Reißfestigkeit um den Faktor 1,7 bis 7,0 auf.

Die gewünschten Eigenschaften lassen sich erzielen, indem nch den üblichen Verfahrensschritten Reinigung und Antigenabspaltung das Material in eine Glycerinlösung eingebracht wird. Hierbei wird dem Material Wasser entzogen. Gleichzeitig kommt es durch Diffusionsvorgänge zur Einlagerung von Glycerin in das Transplantat. Beim nachfolgenden Trocknungsprozeß steigt der prozentuale Glyceringehalt wesentlich an. Gleichartige Wirkungen lassen sich mit Polyäthylenglykol 400 bis 2000 erzielen.

Eindiffundiertes Glycerin oder Polyäthylenglykol wirkt als Schutzfaktor beim Gefrieren. Diese an sich bekannte Tatsache ist jedoch nicht verantwortlich für die erhöhte biologische Stabilität nach einer Implantation, denn es hat sich gezeigt, daß die Gefriertrocknung durch eine Lufttrocknung bei Zimmertemperatur ersetzt werden kann, ohne daß die Resistenz gegen Abbau im lebenden Organismus ungünstig beeinflußt wurde.

Das Verfahren gemäß der Erfindung wird durchgeführt, indem man Skleroproteine wie z. B. Kollagen, Keratin, Elastin und insbesondere Roh-Duren in üblicher Weise wässert, anschließend mit $H_2O_2$ behandelt, danach entfettet, mit Wasser auswäscht, trocknet und sterilisiert, wobei erfindungsgemäß zwischen die Stufe des Auswaschens und die Stufe des Trocknens eine Behandlungsstufe mit Glycerin oder Polyäthylenglykol eingeschoben wird.

Das Glycerin kann in einer 5- bis 50%igen Lösung in Wasser, vorzugsweise als 20—40%ige und insbesondere als 30%ige Glycerinlösung in Wasser verwendet werden.

Das verwendete Polyäthylenglykol hat ein Molekulargewicht von 400 bis 2000 und wird gewöhnlich in einer 5- bis 50%igen Polyäthylenglykollösung in Wasser, insbesondere einer 20- bis 40%igen Lösung verwendet.

Durch das erfindungsgemäße Verfahren ergeben sich folgende Vorteile gegenüber der bisherigen Technik:

Das Produkt ist weich und vor der Anwendung ist keine Rehydrierung erforderlich.

**0 069 156**

Das Produkt ist transparent, z. B. kann man bei Hirnoperationen durch das Transplantat hindurch Liquor und Hirnoberfläche sehen.

Das Produkt weist eine erhöhte biologische Stabilität auf.

Das erfindungsgemäße Verfahren wird nachstehend anhand eines Ausführungsbeispiels zur Herstellung von weicher Trockendura erläutert.

Ausführungsbeispiel

Rohduren, die in konzentrierter NaCl eingelegt geliefert wurden, wurden 24 Stunden gewässert. Danach wurden sie 48 Stunden in 2- bis 20%iges, vorzugsweise 5%iges $H_2O_2$ gelegt. Daraufhin wurden die Duren in einer Soxhlettapparatur in Aceton-Diäthyläther 1 : 1 für eine Zeit von 4 Stunden entfettet. Die entfetteten Duren wurden 12—24 Stunden mit Wasser ausgewaschen.

Die so behandelten Duren wurden 4 Stunden in einer 30%igen Glycerinlösung in Wasser gerührt. Die erhaltenen feuchten Duren wurden in einem Lyophilisator gefriergetrocknet. Alternativ wurden die erhaltenen feuchten Duren bei Zimmertemperatur an der Luft getrocknet.

Nach 12stündigem Trocknen wurden die weichen Trockenduren entnommen und mit 2,5 Mrad sterilisiert.

Die erhaltenen Duren waren weich, transparent und wiesen eine erhöhte biologische Stabilität auf.

Die nach den bisher bekannten Verfahren erhaltenen Duren waren wesentlich härter, nicht transparent und hatten eine niedrigere biologische Stabilität.

Die nachstehenden Daten zeigen in Verbindung mit dem oben angegebenen Diagramm die überraschenden Ergebnisse der Vergleichsversuche.

Tabellarische Übersicht der Reißfestigkeit von Durastreifen
(10 mm breit) nach Implantation, Angaben in kp

(n = 10 pro Gruppe; jeder im Diagramm eingetragene Punkt der Kurve
basiert auf 10 Einzelmessungen)

| Implantations-zeit (Tage) | Lyodura gewonnen durch Gefriertrocknung | Tutoplast gewonnen durch Acetontrocknung | weiche Dura gemäß der Erfindung |
|---|---|---|---|
| 0 | 1,9±0,9<br>100%±47 | 2,0±05<br>105±26 | 3,2±1,3<br>168±68 |
| 1 | 1,9±0,7<br>100%±37 | 1,7±0,3<br>89%±16 | 3,5±0,9<br>184%±47 |
| 4 | 1,4±0,6<br>74%±32 | 1,2±0,4<br>63%±21 | 3,9±0,7<br>205%±37 |
| 7 | 1,4±0,6<br>74%±32 | 0,7±0,3<br>37%±16 | 4,4±1,6<br>232%±84 |
| 14 | 1,3±0,6<br>68%±32 | 0,7±0,4<br>37%±21 | 3,8±1,7<br>200%±90 |
| 21 | 1,0±0,5<br>53%±26 | 0,7±0,3<br>37%±16 | 2,6±1,0<br>137%±53 |
| 31 | 0,9±0,8<br>47%±42 | 0,4±0,6<br>21%±32 | 2,8±1,7<br>147%±90 |

zweite Zeile = Angaben in %
0-Wert Lyodura ≙ 100%.

Die erfindungsgemäß erhaltenen weichen Duren können als Transplantate auf den verschiedensten Gebieten, die dem Fachmann wohlbekannt sind, eingesetzt werden. Eine große Anzahl der Einsatzgebiete wird in der oben genannten Firmenbroschüre auf Seite 2 genannt.

3

**0 069 156**

### Patentansprüche

1. Verfahren zur Herstellung von Skleroproteintransplantaten mit erhöhter biologischer Stabilität durch übliche Behandlung der rohen, von Menschen oder Tieren stammenden Skleroproteine durch Wässern, Behandeln mit $H_2O_2$, Entfetten, Auswaschen, Trocknen und Sterilisieren, dadurch gekennzeichnet, daß man die Skleroproteine nach dem Auswaschen und vor dem Trocknen mit Glycerin oder Polyäthylenglykol behandelt.

2. Verfahren nach Anspruch 1, dadurch gekennzeichnet, daß man eine 5- bis 50%ige Glyzerinlösung in Wasser verwendet.

3. Verfahren nach Anspruch 1, dadurch gekennzeichnet, daß man Polyäthylenglykol mit einem Molekulargewicht von 400 bis 2000 verwendet.

4. Verfahren nach Ansprüchen 1 und 3, dadurch gekennzeichnet, daß man eine 5- bis 50%ige Polyäthylenglykollösung in Wasser verwendet.

5. Verfahren nach Ansprüchen 1—4, dadurch gekennzeichnet, daß man als Skleroproteine vom Menschen oder vom Tier stammendes Kollagen, Keratin, oder Elastin verwendet.

6. Verfahren nach Ansprüchen 1—5, dadurch gekennzeichnet, daß man als Skleroprotein vom Menschen stammende Dura verwendet.

7. Verfahren nach Anspruch 6, dadurch gekennzeichnet, daß man eine 20- bis 40%ige, insbesondere 30%ige Glyzerinlösung in Wasser verwendet.

### Claims

1. A process for preparing sclero protein transplants having an increased biological stability by conventional treatment in which the raw sclero protein from humans or animals is watered, treated with $H_2O_2$, degreased, rinsed, dried and sterilized, characterized in that the sclero protein, after rinsing and prior to drying, is treated with glycerol or polyethylene glycol.

2. The process according to claim 1, characterized in that a 5 to 50% glycerol solution in water is used.

3. The process according to claim 1, characterized in that a polyethylene glycol having a molecular weight of from 400 to 2000 is used.

4. The process according to claims 1 and 3, characterized in that a 5 to 50% polyethylene glycol solution in water is used.

5. The process according to claims 1 to 4, characterized in that collagen, keratin or elastin from humans or animals are used as the sclero protein.

6. The process according to claims 1 to 5, characterized in that dura from humans is used as the sclero protein.

7. The process according to claim 6, characterized in that a 20 to 40%, more specifically a 30%, glycerol solution solution in water is used.

### Revendications

1. Procédé de préparation de transplants (greffes) en scléroprotéines ayant une stabilité biologique améliorée, par traitement usuel des scléroprotéines brutes, d'origine humaine ou animale, par mise en contact avec de l'eau, traitement par $H_2O_2$, dégraissage, lavage, séchage et stérilisation, caractérisé en ce qu'on traite les scléroprotéines, après le lavage et avant le séchage, par du glycérol ou du polyéthylène-glycol.

2. Procédé selon la revendication 1, caractérisé en ce qu'on utilise une solution à 5 à 50% de glycérol dans de l'eau.

3. Procédé selon la revendication 1, caractérisé en ce qu'on utilise du polyéthylène-glycol ayant un poids moléculaire de 400 à 2000.

4. Procédé selon les revendications 1 et 3, caractérisé en ce qu'on utilise une solution à 5 à 50% de polyéthylène-glycol dans de l'eau.

5. Procédé selon les revendications 1 à 4, caractérisé en ce qu'on utilise comme scléroprotéines du collagène, de la kératine ou de l'élastine d'origine humaine ou animale.

6. Procédé selon les revendications 1 à 5, caractérisé en ce qu'on utilise comme scléroprotéine de la dure-mère d'origine humaine.

7. Procédé selon la revendication 6, caractérisé en ce qu'on utilise une solution à 20 à 40%, notamment à 30%, de glycérol dans l'eau.

4

Reißfestigkeit von Durastreifen nach Implantation
Breite 1o mm, n = 1o pro Gruppe
A = acetongetrocknete Dura
B = lyophilisierte Dura
C = weiche Lyodura